# EUROPÄISCHE PATENTSCHRIFT

(11) **EP 0 663 190 B1**
(45) Veröffentlichungstag und Bekanntmachung des Hinweises auf die Patenterteilung: **16.10.1996**
(21) Anmeldenummer: 94101753.5
(22) Anmeldetag: 05.02.1994
(51) Int. Cl.: A61B 17/08

(54) **Vorrichtung zum Verschliessen einer Wunde**
Closure device for wounds
Dispositif pour obturer des plaies

(30) Priorität: 13.01.1994 DE 4400732
(43) Veröffentlichungstag der Anmeldung: 19.07.1995
(73) Patentinhaber: An Haack, Karl Werner, D-44225 Dortmund (DE)
(72) Erfinder: An Haack, Karl Werner, D-44225 Dortmund (DE)
(74) Vertreter: Andrejewski, Walter, Dr.

(56) Entgegenhaltungen:
- DE-A- 3 706 599
- US-A- 3 516 409

## Beschreibung

Die Erfindung betrifft eine Vorrichtung zum Verschließen einer Wunde nach dem Oberbegriff des Patentanspruchs 1, wie sie beispielsweise aus der DE-A-3 706 599 bekannt ist. Die erfindungsgemäße Vorrichtung stellt nicht eine Wundabdeckung, sondern einen Wundverschluß dar, mit dem eine klaffende Wunde, z.B. eine klaffende Operationswunde, zusammengezogen und mit niveaugleichen Wundrändern stoßend geschlossen werden kann. Insoweit ersetzt die Vorrichtung das bekannte Nähen oder Klammern einer solchen Wunde. - Wenngleich die erfindungsgemäße Vorrichtung insbesondere für eine hauptsächlich in gerader Richtung verlaufende Operationswunde geeignet ist, kann nichtsdestoweniger die erfindungsgemäße Vorrichtung auch bei kurvenförmig, z.B. S-förmig oder bogenförmig, geführten Wunden Verwendung finden, unabhängig davon, ob es sich um Klaff-, Schnitt-, Riß- oder Quetschwunden im Operations- sowie im Gelegenheitswundenbereich handelt.

Die Erfindung geht aus von einer bekannten Vorrichtung des eingangs beschriebenen Aufbaus sowie der eingangs beschriebenen Zweckbestimmung (DE 37 06 599 C2). Bei dieser Ausführungsform ist der Reißverschluß in bezug auf die Verschlußgliederreihen und die Tragbänder ein für textile Kleidungsstücke üblicher, flexibler Reißverschluß mit dehnungsarmen Tragbändern und im gekuppelten Zustand stauchfesten Verschlußgliederreihen aus Polyester oder Polyamid. Auf der der Wunde zugeordneten Unterseite sind mit Abstand von den Verschlußgliederreihen auf den Tragbändern wundfreiraumbildende Distanzstreifen angeordnet. Die Einrichtung zur Klebverbindung besteht aus mit Tragbändern verbundenen, mit einem Überstand über die Tragbänder überstehenden Klebestreifen mit hautseitiger, hautfreundlicher Klebeschicht. Die Tragbänder sind von dem Klebestreifen zumindest über einen Teil ihrer Breite unterlegt. Die Klebestreifen einschließlich der Überstände weisen eine Breite auf, die zur Aufnahme der Querzugkräfte bei durch den Reißverschluß geschlossener Wunde mit stoßenden, d.h. gegeneinandergedrückten Wundrändern, geeignet ist. Die bekannte Vorrichtung hat sich bewährt und genügt in medizinischer Hinsicht allen Anforderungen. Sie ist jedoch in fertigungstechnischer Hinsicht aufwendig, was insbesondere deshalb stört, weil es sich bei diesen Vorrichtungen um ausgesprochene Massenprodukte handelt.

Der Erfindung liegt das technische Problem zugrunde, eine Vorrichtung des eingangs beschriebenen Aufbaus sowie der eingangs beschriebenen Zweckbestimmung zu schaffen, die sich sehr einfach fertigen läßt und insbesondere eine flexible industrielle Massenanfertigung zuläßt.

Zur Lösung dieser Aufgabe ist Gegenstand der Erfindung eine Vorrichtung zum Verschließen einer Wunde, insbesondere einer hauptsächlich in gerader Richtung verlaufenden Operationswunde, die einen aus zwei textilen Tragbändern und daran angeordneten Verschlußgliederreihen bestehenden Reißverschluß mit Reißverschlußschieber und zwei wird freiraumbildende Distanzstreifen aufweist und längs der Wundränder auf die Haut des Patienten aufklebbar ist, wobei die Kombination der folgenden Merkmale verwirklicht ist:
a) die Tragbänder des Reißverschlusses sind um eine Längsfaltkante zu einem Doppeltragbandbereich mit an die Verschlußgliederreihen angeschlossenem Tragbandstreifen und einem Umschlagtragbandstreifen umgelegt,
b) je ein wundfreiraumbildender Distanzstreifen ist zwischen den an die Verschlußgliederreihen angeschlossenen Tragbandstreifen und dem Umschlagtragbandstreifen ist ein angeordnet,
c) auf die freiliegende Oberfläche des Umschlagtragbandstreifens ist unmittelbar oder mittelbar ein hautfreundlicher Kleber aufgebracht,
wobei der an die Verschlußgliederreihen angeschlossene Tragbandstreifen, der Umschlagtragbandstreifen und der Distanzstreifen im Doppeltragbandbereich miteinander vereinigt sind.

Um die Querzugkräfte aufzunehmen, die beim Verschließen einer klaffenden Wunde durch Schließen des beim Aufkleben auf die Haut des Patienten längs der beiden Wundränder verlaufenden Reißverschlußstreifens auftreten, muß eine ausreichende Breite der klebenden Fläche eingerichtet werden. Dazu lehrt die Erfindung, daß die Tragbänder des Reißverschlusses jeweils eine Breite von mehreren Zentimetern aufweisen und in Breitenmitte die Längsfaltkante angeordnet ist. Vorzugsweise ist, darüber hinausgehend, die Anordnung so getroffen, daß die mit der Klebeschicht versehenen Umschlagtragbandstreifen eine Breite von mehreren Zentimetern besitzen.

Die Erfindung geht von der Erkenntnis aus, daß ein üblicher Reißverschluß unschwer und ohne große Änderung der Fertigungstechnik mit Tragbändern versehen werden kann, die eine ausreichende Breite aufweisen, um, mittelbar oder unmittelbar mit einem Kleber versehen, die vorstehend schon angedeuteten, beim Schließen einer Wunde durch Schließen des Reißverschlusses auftretenden Querzugkräfte aufzunehmen. Insoweit verwendet die Erfindung mit den Reißverschlüssen, deren Tragbänder in der beschriebenen Art und Weise eingerichtet ist, Reißverschlüsse, die mit den üblichen Fertigungseinrichtungen für Reißverschlüsse unschwer gefertigt werden können. Entsprechende Bandwebautomaten oder Bandwirkautomaten sind umfangreich bekannt und bewährt. Die weiteren Arbeitsschritte, die erforderlich sind, um aus diesem Vorprodukt in Form eines Reißverschlusses die erfindungsgemäße Vorrichtung herzustellen, sind einfach und können sowohl auf einfache Weise von Hand als auch auf einfache Weise automatisch hergestellt werden.

Im einzelnen bestehen im Rahmen der Erfindung mehrere Möglichkeiten der weiteren Ausbildung und Gestaltung. Da die wundfreiraumbildenden Distanzstreifen im Bereich der Verschlußgliederreihen den Wundfreiraum definieren, empfiehlt es sich, die wundfreiraumbildenden Distanzstreifen mit etwa der Breite des Doppelbandbereiches auszurüsten und als Einlagen auszuführen. Die Distanzstreifen können gewebt, gewirkt oder aus nonwoven Vliesstreifen ausreichender Dichte gefertigt sein, wobei Naturfasern oder Kunststoffasern bzw. Kunststoffilamente eingesetzt werden können.

Der hautfreundliche Kleber kann auf verschiedene Weise angebracht werden. Bei einer Ausführungsform einer erfindungsgemäßen Vorrichtung ist der hautfreundliche Kleber als Heftpflasterkleber ausgeführt und unmittelbar auf die freiliegende Oberfläche der Umschlagtragbandstreifen aufgebracht. Es besteht aber auch die Möglichkeit, die Anordnung so zu treffen, daß der hautfreundliche Kleber auf ein Heftpflasterklebeband aufgebracht und dieses gegen die freiliegende Oberfläche der Umschlagtragbandstreifen angelegt sowie mit den Umschlagtragbandstreifen verbunden ist. Wenn große Querzugbeanspruchungen aufzunehmen sind, weil es sich beispielsweise um weitklaffende Wunden handelt, lehrt die Erfindung, daß das Heftpflasterklebeband auf der den Verschlußgliederreihen abgewandten Seite über die Umschlagtragbandstreifen vorsteht.

Die Verbindung der beschriebenen Bauteile miteinander kann auf verschiedene Weise erfolgen, beispielsweise auch durch Kleben. Nach einer bevorzugten Ausführungsform der Erfindung sind der Tragbandstreifen, der wundfreiraumbildende Distanzstreifen und der Umschlagtragbandstreifen im Doppelbandbereich durch Nähnähte und/oder Heftnähte miteinander vereinigt.

Bei einer anderen Ausführungsform sind der Tragbandstreifen, der wundfreiraumbildende Distanzstreifen, der Umschlagtragbandstreifen sowie das Heftpflasterklebeband an jeder Längsseite des Reißverschlusses im Doppelbandbereich durch Nähnähte und/oder Heftnähte miteinander vereinigt. Aus bedienungstechnischen Gründen beim Verschließen einer Wunde empfiehlt es sich, an die Tragbänder anfangsseitig und endseitig Halteschlaufen anzuschließen.

Im folgenden wird die Erfindung anhand einer lediglich ein Ausführungsbeispiel darstellenden Zeichnung ausführlicher erläutert. Es zeigen in schematischer Darstellung
- Fig. 1: eine Draufsicht auf eine erfindungsgemäße Vorrichtung,
- Fig. 2: einen Schnitt in Richtung A-A durch den Gegenstand der Fig. 1,
- Fig. 3: entsprechend der Fig. 2 einen Schnitt durch eine andere Ausführungsform der Vorrichtung,
- Fig. 4: entsprechend der Fig. 2 einen Schnitt durch eine weitere Ausführungsform der Vorrichtung,
- Fig. 5: entsprechend der Fig. 2 einen Schnitt durch eine nochmals andere Ausführungsform, und
- Fig. 6: eine Draufsicht auf eine erfindungsgemäße Vorrichtung mit geöffnetem Reißverschluß beim Verschließen einer Operationswunde mit Hilfe dieser Vorrichtung.

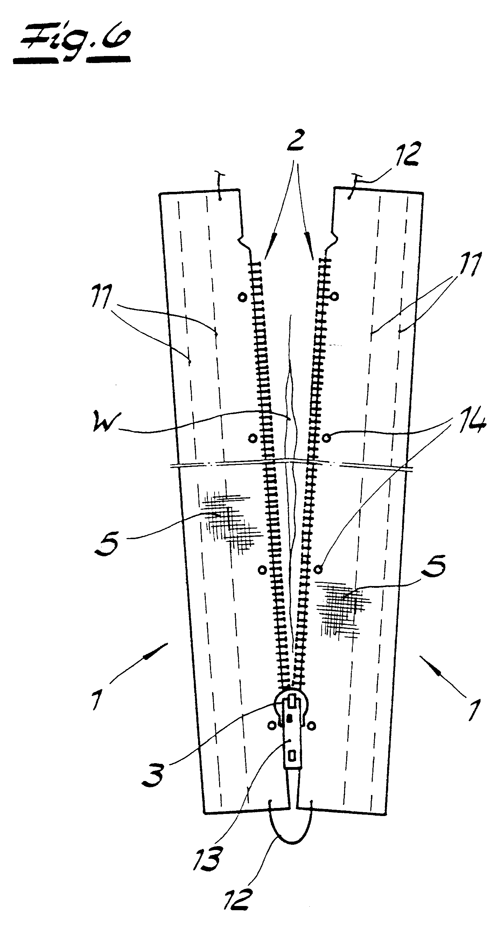

Die in den Figuren dargestellte Vorrichtung dient zum Verschliessen einer Wunde, insbesondere einer hauptsächlich in gerader Richtung verlaufenden Operationswunde W, die einen aus zwei textilen Tragbändern 1 und daran angeordneten Verschlußgliederreihen 2 bestehenden Reißverschluß mit Reißverschlußschieber 3 aufweist und längs der Wundränder auf die Haut eines Patienten aufklebbar ist. Insoweit wird auf die Fig. 6 verwiesen.

Aus einer vergleichenden Betrachtung, insbesondere der Fig. 2 bis 5, entnimmt man, daß die Tragbänder 1 des Reißverschlusses um eine Längsfaltkante 4 zu einem Doppelbandbereich mit an die Reißverschlußgliederreihen 2 angeschlossenem Tragbandstreifen 5 und einem Umschlagtragbandstreifen 6 umgelegt sind. Zwischen dem an die Reißverschlußgliederreihen 2 angeschlossenen Tragbandstreifen 5 und dem Umschlagtragbandstreifen 6 ist ein wundfreiraumbildender Distanzstreifen 7 angeordnet. Auf die freiliegende Oberfläche des Umschlagtragbandstreifens 6 ist unmittelbar oder mittelbar ein hautfreundlicher Kleber 8 aufgebracht. Die Anordnung ist so getroffen, daß der an die Reißverschlußgliederreihen 2 angeschlossene Tragbandstreifen 5, der Umschlagtragbandstreifen 6 und der Distanzstreifen 7 im Doppelbandbereich miteinander vereinigt sind.

Die Tragbänder 1 des Reißverschlusses mögen jeweils eine Breite von mehreren Zentimetern aufweisen, in Breitenmitte ist die Längsfaltkante 4 angeordnet. Wenn große Querzugkräfte aufzunehmen sind, wird man die Anordnung so treffen, daß die mit dem Kleber 8 versehenen Umschlagtragbandstreifen 6 eine Breite von mehreren Zentimetern aufweisen.

Die wundfreiraumbildenden Distanzstreifen 7, die insbesondere im Bereich der Wunde W wirken müssen, besitzen, wie in den Zeichnungen dargestellt, vorzugsweise etwa die Breite des Doppelbandbereiches. Sie sind als textile Einlagen ausgeführt. Sie können gewebt, gewirkt oder als nonwoven-Vliesstreifen ausreichender Dichte ausgeführt sein.

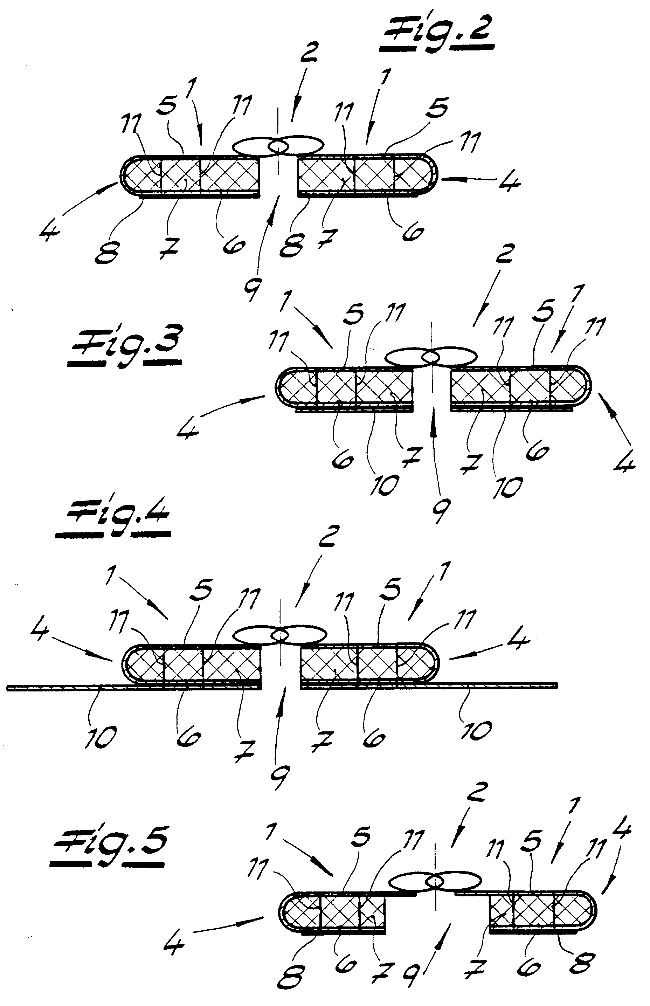

Nach bevorzugter Ausführungsform der Erfindung ist der hautfreundliche Kleber 8 als Heftpflasterkleber ausgeführt, jedoch unmittelbar auf die freiliegende Oberfläche der Umschlagtragbandstreifen 6 aufgebracht. Insoweit wird auf die Fig. 2 und 5 verwiesen. Aus einer vergleichenden Betrachtung dieser beiden Figuren erkennt man gleichzeitig, daß der sogenannte Wundfreiraum 9 in bezug auf seine Breite unterschiedlich eingerichtet sein kann, wobei es lediglich erforderlich ist, den Umschlagtragbandstreifen 6 und den Distanzstreifen 7 breiter oder weniger breit auszuführen. Der hautfreundliche Kleber 8 kann aber auch auf ein Heftpflasterklebeband 10 aufgebracht werden. Dazu wird auf die Fig. 3 und 4 verwiesen. Man erkennt, daß das Heftpflasterklebeband 10 gegen die freiliegende Oberfläche der Umschlagtragbandstreifen 6 angelegt sowie mit den Umschlagtragbandstreifen 6 verbunden ist. Ohne weiteres kann, wenn sehr große Querzugkräfte aufzunehmen sind, die Anordnung so getroffen werden, daß das Heftpflasterklebeband 10 auf der den Verschlußgliederreihen 2 abgewandten Seite an jeder Reißverschlußseite über die Umschlagtragbandstreifen 4 vorsteht. Das verdeutlicht die Fig. 4. Die Breite des Überstandes richtet sich nach den aufzunehmenden Zugbeanspruchungen.

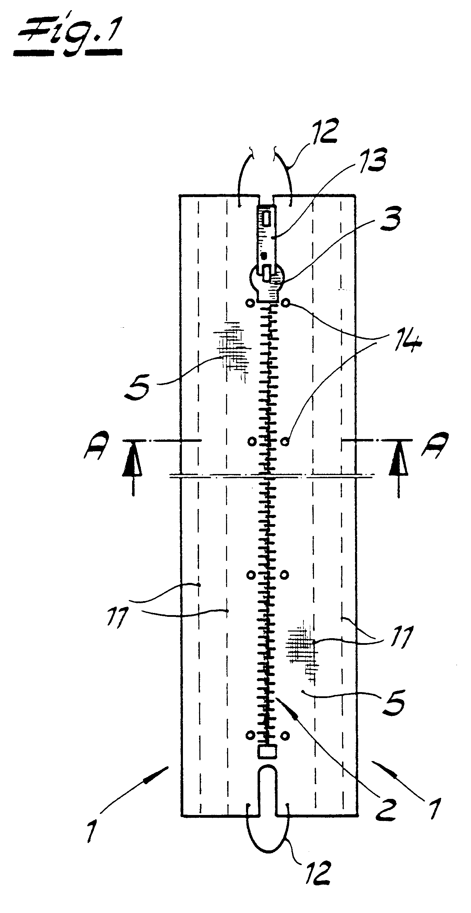

In den Figuren wurde angedeutet, daß die Tragbandstreifen 1, der wundfreiraumbildende Distanzstreifen 7 und der Umschlagtragbandstreifen 6 im Doppelbandbereich durch Nähnähte und/oder Heftnähte 11 miteinander vereinigt sind. Bei der Ausführungsform mit Heftpflasterklebebändern 10 wird man die Tragbandstreifen 1, die wundfreiraumbildenden Distanzstreifen 7, die Umschlagtragbandstreifen 6 sowie die Heftpflasterklebebänder 10 im Doppelbandbereich durch Nähnähte oder Heftnähte 11 miteinander verbinden, wie es die Fig. 3 und 4 andeuten. In den Fig. 1 und 6 wurde angedeutet, daß in die Tragbänder 1 anfangsseitig und endseitig Halteschlaufen 12 angeschlossen sind. Beim Schließen des Reißverschlusses, einem Vorgang, bei dem die Ränder der zunächst klaffenden Wunde W gleichsam stoßend niveaugleich aneinandergezogen werden, greift der Operateur einerseits an dem Reißverschlußschieber 3 mit seinem Handgriff 13 und andererseits an der am geschlossenen Ende des Reißverschlusses angebrachten Halteschlaufe 12 an. Der Reißverschlußschieber 3 wird so weit schiebend bewegt, bis der Reißverschluß vollständig geschlossen ist, wobei die Wundränder wie beschrieben zueinander hingezogen werden. Wird der Reißverschluß geöffnet, so wird man umgekehrt am offenen Ende des Reißverschlusses die Halteschlaufen 12 festhalten und den Reißverschlußschieber 3 in Öffnungsrichtung bewegen. Das kann beispielsweise zur Kontrolle des Heilungsprozesses an der Wunde W erforderlich sein. Jedenfalls kann mit Hilfe der Halteschlaufen 12 sichergestellt werden, daß die Zugkräfte, die auftreten, wenn der Reißverschluß mit Hilfe des Reißverschlußschiebers 3 geschlossen wird oder aber wieder geöffnet wird, nicht über den Kleber 8 von der Haut des Patienten aufgenommen werden müssen, sondern vielmehr von den Händen des Operateurs, die einerseits am Reißverschlußschieber 3 und andererseits an einer zur Aufnahme der Zugkräfte eingerichteten Halteschlaufe 12 angreifen. Es versteht sich, daß die Tragbänder 1 des Reißverschlusses, die wundfreiraumbildenden Distanzstreifen 7 und gegebenenfalls der Kleber 8 bzw. die Heftpflasterklebebänder 10 Lüftungslöcher 14 in Form von Ausstanzungen aufweisen können.

## Patentansprüche

1. Vorrichtung zum Verschließen einer Wunde, insbesondere einer hauptsächlich in gerader Richtung verlaufenden Operationswunde, die einen aus zwei textilen Tragbändern (1) und daran angeordneten Verschlußgliederreihen bestehenden Reißverschluß mit Reißverschlußschieber (3) und zwei wundfreiraumbildende Distanzstreifen (7) aufweist und längs der Wundränder auf die Haut des Patienten aufklebbar ist, dadurch gekennzeichnet, daß bei der Vorrichtung die Kombination der folgenden Merkmale verwirklicht ist:
a) die Tragbänder (1) des Reißverschlusses sind um eine Längsfaltkante (4) zu einem Doppeltragbandbereich mit an die Verschlußgliederreihen angeschlossenem Tragbandstreifen (5) und einem Umschlagtragbandstreifen (6) umgelegt,
b) je ein wundfreiraumbildender Distanzstreifen (7) ist zwischen den an die Verschlußgliederreihen angeschlossenen Tragbandstreifen (5) und dem Umschlagtragbandstreifen (6) angeordnet,
c) auf die freiliegende Oberfläche des Umschlagtragbandstreifens (6) ist unmittelbar oder mittelbar ein hautfreundlicher Kleber aufgebracht,
wobei der an die Verschlußgliederreihen angeschlossene Tragbandstreifen (5), der Umschlagtragbandstreifen (6) und der Distanzstreifen (7) im Doppeltragbandbereich miteinander vereinigt sind.

2. Vorrichtung nach Anspruch 1, wobei die Tragbänder (1) des Reißverschlusses jeweils eine Breite von mehreren Zentimetern aufweisen und in Breitenmitte die Längsfaltkante angeordnet ist.

3. Vorrichtung nach einem der Ansprüche 1 oder 2, wobei die mit der Klebeschicht versehenen Umschlagtragbandstreifen (6) eine Breite von mehreren Zentimetern aufweisen.

4. Vorrichtung nach einem der Ansprüche 1 bis 3, wobei die wundfreiraumbildenden Distanzstreifen (7) etwa die Breite des Doppelbandbereiches aufweisen und als textile Einlagen ausgeführt sind.

5. Vorrichtung nach einem der Ansprüche 1 bis 4, wobei der hautfreundliche Kleber als Heftpflasterkleber ausgeführt und unmittelbar auf die freiliegende Oberfläche der Umschlagtragbandstreifen (6) aufgebracht ist.

6. Vorrichtung nach einem der Ansprüche 1 bis 4, wobei der hautfreundliche Kleber auf ein Heftpflasterklebeband aufgebracht und dieses gegen die freiliegende Oberfläche der Umschlagtragbandstreifen (6) angelegt sowie mit den Umschlagtragbandstreifen (6) verbunden ist.

7. Vorrichtung nach Anspruch 6, wobei das Heftpflasterklebeband auf der den Verschlußgliederreihen (2) abgewandten Seite über die Umschlagtragbandstreifen (6) vorsteht.

8. Vorrichtung nach einem der Ansprüche 1 bis 6, wobei der Tragbandstreifen (1), der wundfreiraumbildende Distanzstreifen (7) und der Umschlagtragbandstreifen (6) im Doppelbandbereich an jeder Längsseite des Reißverschlusses durch Nähnähte und/oder Heftnähte (11) miteinander vereinigt sind.

9. Vorrichtung nach Anspruch 7, wobei der Tragbandstreifen (1), der wundfreiraumbildende Distanzstreifen (7), der Umschlagtragbandstreifen (6) sowie das Heftplasterklebeband an jeder Längsseite des Reißverschlusses im Doppelbandbereich durch Nähnähte und/oder Heftnähte (11) miteinander vereinigt sind.

10. Vorrichtung nach einem der Ansprüche 1 bis 9, wobei an die Tragbänder anfangsseitig und endseitig Halteschlaufen (12) angeschlossen sind.

## Claims

1. A device for the closure of a wound, particularly an operation wound running mainly in a straight direction, which comprises a sliding clasp fastener, consisting of two textile stringer tapes (1) and rows of closure elements disposed thereon and having a sliding clasp fastener sliding clasp (3) and two distance strips (7) forming a free space for the wound, which device can be stuck to the skin of the patient along the edges of the wound, characterised in that the combination of the following features is put into effect in the device:
a) the stringer tapes (1) of the sliding clasp fastener are folded round a longitudinal folding edge (4) to form a double stringer tape region having a stringer tape strip (5) attached to the rows of closure elements and a backing stringer tape strip (6),
b) a distance strip (7) forming a free space for the wound is disposed in each case between the stringer tape strip (5) attached to the rows of closure elements and the backing stringer tape strip (6),
c) an adhesive which is kind to the skin is applied directly or indirectly to the exposed surface of the backing stringer tape strip (6),
wherein the stringer tape strip (5) attached to the rows of closure elements, the backing stringer tape strip (6) and the distance strips (7) are joined to each other in the double stringer tape region.

2. A device according to claim 1, wherein the stringer tapes (1) of the sliding clasp fastener each have a width of several centimetres and the longitudinal folding edge is disposed in the centre of their width.

3. A device according to either one of claims 1 or 2, wherein the backing stringer tape strips (6) provided with the adhesive layer have a width of several centimetres.

4. A device according to any one of claims 1 to 3, wherein the distance strips (7) forming the free space for the wound are approximately the width of the double strip region and are constructed as textile inserts.

5. A device according to any one of claims 1 to 4, wherein the adhesive which is kind to the skin is constructed as a sticking plaster adhesive and is applied directly to the exposed surface of the backing stringer tape strip (6).

6. A device according to any one of claims 1 to 4, wherein the adhesive which is kind to the skin is applied to a sticking plaster adhesive tape and the latter is placed against the exposed surface of the backing stringer tape strip (6) and is joined to the backing stringer tape strip (6).

7. A device according to claim 6, wherein the sticking plaster adhesive strip protrudes beyond the backing stringer tape strip (6) on the side remote from the rows of closure elements (2).

8. A device according to any one of claims 1 to 6, wherein the stringer tape strip (1), the distance strip (7) forming a free space for the wound, and the backing stringer tape strip (6) are joined to each other in the double strip region on each longitudinal side of the sliding clasp fastener by sewn seams and/or tacked seams (11).

9. A device according to claim 7, wherein the stringer tape strip (1), the distance strip (7) forming a free space for the wound, the backing stringer tape strip (6) and the sticking plaster adhesive strip are joined to each other in the double strip region on each longitudinal side of the sliding clasp fastener by sewn seams and/or tacked seams (11).

10. A device according to any one of claims 1 to 9, wherein holding loops (12) are attached to the start and end of the stringer tapes.

## Revendications

1. Dispositif pour fermer une plaie, en particulier une plaie opératoire orientée principalement dans un sens rectiligne, qui présente une fermeture éclair avec un curseur (3) et deux bandes d'écartement (7) créant un espace libre sur la plaie, consistant en deux bandes de support (1) en textile et des séries de maillons de fermeture disposés dessus, et qui peut être collé le long des lèvres de la plaie sur la peau du patient, caractérisé en ce que dans le dispositif la combinaison des caractéristiques suivantes est mise en oeuvre :
a) les bandes de support (1) de la fermeture éclair sont repliées au droit d'un bord à pli en longueur (4) sur une zone à bande de support double avec le ruban (5) de bande de support attaché aux séries de maillons de fermeture et un ruban de bande de support d'enveloppe (6),
b) chaque bande d'écartement (7) créant un espace libre sur la plaie est disposée entre le ruban de bande de support (5) attaché aux séries de maillons de fermeture et le ruban de bande de support d'enveloppe (6),
c) sur la surface dégagée du ruban de bande de support d'enveloppe (6) est introduit directement ou indirectement un adhésif dermophile,
le ruban (5) de bande de support attaché aux séries de maillons de fermeture, le ruban de bande de support d'enveloppe (6) et la bande d'écartement (7) étant unis les uns avec les autres dans une zone à bande de support double.

2. Dispositif selon la revendication 1, dans lequel les bandes de support (1) de la fermeture éclair présentent, à chaque fois, une largeur de plusieurs centimètres et dans lequel le bord à pli en longueur est disposé au centre de la largeur.

3. Dispositif selon la revendication 1 ou 2, dans lequel les rubans de bande de support d'enveloppe (6) munis d'une couche d'adhésif présentent une largeur de plusieurs centimètres.

4. Dispositif selon l'une des revendications 1 à 3, dans lequel les bandes d'écartement (7) créant un espace libre sur la plaie présentent environ la largeur de la zone à bande de support double et sont réalisées en tant que garnitures textiles.

5. Dispositif selon l'une des revendications 1 à 4, dans lequel l'adhésif dermophile est réalisé comme un adhésif de sparadrap et appliqué indirectement sur la surface dégagée du ruban de bande de support d'enveloppe (6).

6. Dispositif selon l'une des revendications 1 à 4, dans lequel l'adhésif dermophile est appliqué sur une bande adhésive de sparadrap et dans lequel cette bande est placée contre la surface dégagée du ruban de bande de support d'enveloppe (6), de manière à être reliée au ruban de bande de support d'enveloppe (6).

7. Dispositif selon la revendication 6, dans lequel la bande adhésive de sparadrap déborde du côté opposé aux séries de maillons de fermeture (2) sur le ruban de bande de support d'enveloppe (6).

8. Dispositif selon l'une des revendications 1 à 6, dans lequel le ruban de bande de support (1), la bande d'écartement (7) créant un espace libre sur la plaie et le ruban de bande de support d'enveloppe (6) sont unis les uns avec les autres dans la zone à bande de support double de chaque côté long de la fermeture éclair par couture et/ou faufilure (11).

9. Dispositif selon la revendication 7, dans lequel le ruban de bande de support (1), la bande d'écartement (7) créant un espace libre sur la plaie, le ruban de bande de support d'enveloppe (6), ainsi que la bande adhésive de sparadrap, sont unis les uns avec les autres de chaque côté long de la fermeture éclair dans la zone à bande de support double par couture et/ou faufilure (11).

10. Dispositif selon l'une des revendications 1 à 9, dans lequel une dragonne (12) est attachée au début et à la fin des bandes de support.
